# FASCICULE DE BREVET EUROPEEN

(11) **EP 3 077 355 B1**
(45) Date de publication et mention de la délivrance du brevet: **07.02.2018**
(21) Numéro de dépôt: 14821796.1
(22) Date de dépôt: 04.12.2014
(51) Int. Cl.: C07C 45/45, C07C 45/65, C07C 47/575, C07C 309/65, C07C 309/66, C07C 309/73, C07C 233/18, C07C 303/28

(54) **NOUVEAU PROCEDE DE SYNTHESE DU 7-METHOXY-NAPHTALENE-1-CARBALDEHYDE ET APPLICATION A LA SYNTHESE DE L'AGOMELATINE**
NEUARTIGES VERFAHREN ZUR SYNTHESE VON 7-METHOXY-NAPHTHALEN-1-CARBALDEHYD UND VERWENDUNG BEI DER SYNTHESE VON AGOMELATIN
NOVEL METHOD FOR THE SYNTHESIS OF 7-METHOXY-NAPHTHALENE-1-CARBALDEHYDE AND USE THEREOF IN THE SYNTHESIS OF AGOMELATINE

(30) Priorité: 05.12.2013 FR 1362200
(43) Date de publication de la demande: 12.10.2016
(73) Titulaire: Les Laboratoires Servier, 92284 Suresnes (FR)
(72) Inventeur: BRIERE, Jean-François, 76920 Amfreville-la-Mi-Voie (FR); LEBEUF, Raphaël, 59000 Lille (FR); LEVACHER, Vincent, 76160 Fontaine-sous-Preaux (FR); HARDOUIN, Christophe, 76310 Sainte Adresse (FR); LECOUVE, Jean-Pierre, 76600 Le Havre (FR)
(86) Numéro de dépôt international: PCT/FR2014/053159
(87) Numéro de publication internationale: WO 2015/082849

(56) Documents cités:
- FR-A1- 2 918 369
- GARIGIPATI R S ET AL: "Novel frameworks for trifluoromethyl ketone and phosphonate TSA inhibitors of type II PLA2", BIOORGANIC & MEDICINAL CHEMISTRY LETTERS, PERGAMON, AMSTERDAM, NL, vol. 7, no. 11, 3 juin 1997 (1997-06-03), pages 1421-1426, XP004136229, ISSN: 0960-894X, DOI: 10.1016/S0960-894X(97)00246-1
- KANDAGATLA, B. ET AL: "A facile synthesis of melatonergic antidepressant agomelatine", TETRAHEDRON LETTERS, vol. 53, 26 octobre 2012 (2012-10-26), pages 7125-7127, XP002727615, cité dans la demande

## Description

La présente invention concerne un nouveau procédé de synthèse industriel du 7-méthoxy-naphtalène-1-carbaldéhyde et son application à la production industrielle de l agomélatine ou *N*-[2-(7-méthoxy-1-naphtyl)éthyl]acétamide.

Plus spécifiquement, la présente invention concerne un procédé de synthèse industriel du composé de formule (I) :

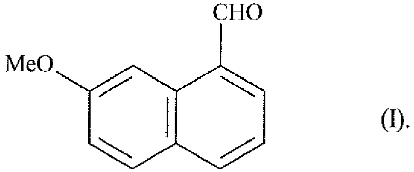

Le composé de formule (I) obtenu selon le procédé de l'invention est utile dans la synthèse de l'agomélatine ou *N*-[2-(7-méthoxy-1-naphtyl)éthyl]acétamide de formule (II) :

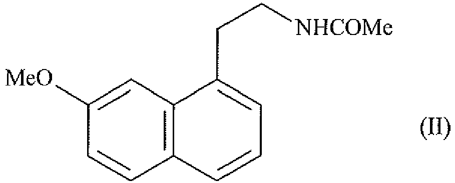

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

Compte-tenu de l'intérêt pharmaceutique de ce composé, il était important de pouvoir y accéder avec un procédé de synthèse performant, facilement transposable à l'échelle industrielle, conduisant à l'agomélatine avec un bon rendement et une excellente pureté, à partir de matières premières bon marché et aisément accessibles.

Le brevet EP 0 447 285 décrit l'accès en huit étapes à l'agomélatine à partir de la 7-méthoxy-1-tétralone. Cependant, transposé à l'échelle industrielle, il a été rapidement mis en évidence des difficultés de mise en oeuvre de ce procédé.

La littérature décrit l'accès en 5 étapes au 7-méthoxy-naphtalène-1-carbaldéhyde à partir de la 8-amino-naphtalén-2-ol (Kandagatla et al. Tetrahedron Lett. 2012, 53, 7125-7127) ou en 3 étapes à partir du 2-(7-methoxy-1-naphtyl)éthanol (FR 2 918 369). Il a été aussi décrit la préparation en 4 étapes du 7-méthoxy-naphtalène-1-carbaldéhyde à partir de la 7-méthoxy-tétralone (Garipati et al. Bioorg. Med. Chem. Lett. 1997, 7, 1421-1426). Cependant, la 7-méthoxy-1-tétralone et la 8-amino-naphtalén-2-ol sont des matières premières onéreuses et, par conséquent, la recherche de nouvelles voies de synthèse, en particulier à partir de réactifs moins chers, est toujours d'actualité.

La demanderesse a poursuivi ses investigations et a mis au point une nouvelle synthèse industrielle qui conduit, de façon reproductible et sans nécessiter de purification laborieuse, à l'agomélatine avec une pureté qui est compatible avec son utilisation comme principe actif pharmaceutique, à partir d'une matière première moins onéreuse et plus facilement accessible.

En particulier, la demanderesse a présentement mis au point un nouveau procédé de synthèse industriel permettant d'obtenir le 7-méthoxy-naphtalène-1-carbaldéhyde de façon reproductible et sans nécessiter de purification laborieuse, en utilisant comme matière première le 7-méthoxy-naphtalén-2-ol. Cette nouvelle matière première présente l'avantage d'être simple, aisément accessible en grandes quantités avec des coûts moindres. Le 7-méthoxy-naphtalén-2-ol présente également l'avantage de posséder dans sa structure un noyau naphtalène, ce qui évite d'intégrer dans la synthèse une étape d'aromatisation, étape toujours délicate d'un point de vue industriel.

Plus spécifiquement, la présente invention concerne un procédé de synthèse industriel du composé de formule (I) : 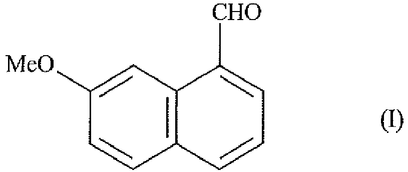 caractérisé en ce que l'on met en réaction le 7-méthoxy-naphtalén-2-ol de formule (III) : 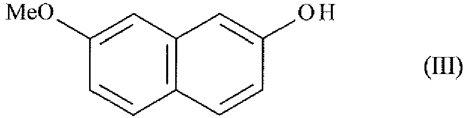 sur lequel on réalise une réaction de formylation sur la position 1 du composé de formule (III) pour conduire au composé de formule (IV) : 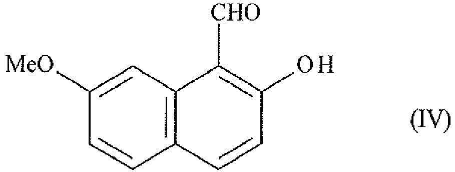 composé de formule (IV) qui est soumis à une réaction de sulfonylation pour conduire au composé de formule (V) : 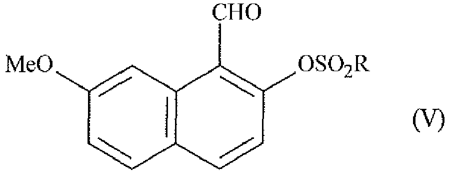 dans laquelle R représente un groupement -CH₃, -(CH₂)₂-CH₃, -CF₃ ou toluyle ;
composé de formule (V) qui subit une réaction de désoxygénation en présence d'un métal de transition et d'un agent réducteur pour conduire au composé de formule (I) que l'on isole sous forme d'un solide.

Le composé de formule (III) est commercial ou aisément accessible à l'homme du métier par des réactions de chimie classiques ou décrites dans la littérature.

R représente préférentiellement un groupement -CH₃ ou toluyle.

Dans le procédé selon l'invention, la transformation du composé de formule (III) en composé de formule (IV) consiste en l'action de l'orthoformiate d'éthyle en présence d'aniline suivie d'une hydrolyse de l'imine intermédiaire obtenue.

Dans le procédé selon l'invention, la transformation du composé de formule (IV) en composé de formule (V) consiste en une étape de sulfonylation réalisée grâce à l'action d'un chlorure de sulfonyle, d'un anhydride sulfonique ou d'un sulfonimide. Selon un mode de réalisation préféré, cette étape de sulfonylation est réalisée grâce à l'action d'un chlorure de sulfonyle et, en particulier, le chlorure de tosyle ou le chlorure de mésyle.

Dans le procédé selon l'invention, la transformation du composé de formule (V) en composé de formule (I) consiste en une étape de désoxygénation en présence d'un métal de transition et d'un agent réducteur.
De manière préférée, le métal de transition est le nickel, le palladium ou le platine. Le métal de transition peut être soit sous forme d'un sel ou soit sous forme d'un corps simple.

Préférentiellement, le sel de métal de transition est un sel de nickel ou un sel de palladium, plus préférentiellement, un sel de palladium.
Avantageusement, l'agent réducteur est soit un hydrure tel que le borohydrure de sodium ou l'hydrure de lithium et d'aluminium ; soit un aminoborane tel que le diméthylamine-borane ; soit un alkoxysilane tel que le diméthoxyméthylsilane ; soit un alkylsilane tel que le triéthylsilane ; soit un métal alcalino-terreux tel que le magnésium ; soit le dihydrogène. De manière préférée, l'agent réducteur est le dihydrogène qui est utilisé directement sous sa forme gazeuse ou bien est indirectement obtenu par décomposition d'un formiate d'ammonium. L'agent réducteur est préférentiellement le dihydrogène obtenu par décomposition d'un formiate d'ammonium.
Selon un autre mode de réalisation préféré, la transformation du composé de formule (V) en composé de formule (I) consiste en une étape de désoxygénation en présence de nickel, en particulier un sel de nickel, et d'un hydrure, préférentiellement le borohydrure de sodium.
Selon un autre mode de réalisation préféré, la transformation du composé de formule (V) en composé de formule (I) consiste en une étape de désoxygénation en présence de palladium et de dihydrogène.
Selon un autre mode de réalisation préféré, la transformation du composé de formule (V) en composé de formule (I) consiste en une étape de désoxygénation en présence de palladium et d'un métal alcalino-terreux, préférentiellement, le magnésium. Avantageusement, la réaction de transformation du composé de formule (V) en composé de formule (I) est réalisée dans le diméthylformamide, le dioxane, le tétrahydrofurane et le toluène et, plus préférentiellement, le diméthylformamide.
De façon préférentielle, la réaction de transformation du composé de formule (V) en composé de formule (I) est réalisée entre 25 °C et 110°C, plus particulièrement, entre 40 °C et 95 °C.
Selon un autre mode de réalisation préféré, la transformation du composé de formule (V) en composé de formule (I) consiste en une étape de désoxygénation en présence d'un métal de transition, d'un agent réducteur et d'un ligand.
Le ligand peut être soit un ligand phosphine, soit un ligand diaminocarbène, plus préférentiellement, un ligand phosphine et, plus particulièrement, le 1,3-bis(diphénylphosphino)propane ou le (9,9-diméthyl-9*H*-xanthène-4,5-diyl)bis (diphénylphosphane).

Une variante avantageuse au procédé de synthèse industriel consiste en ce que l'on réalise directement la transformation du composé de formule (IV) en composé de formule (I), ladite réaction de sulfonylation et ladite réaction de désoxygénation en présence d'un métal de transition se faisant en « one-pot ».

Ce procédé est particulièrement intéressant pour les raisons suivantes :
- il permet d'obtenir à l'échelle industrielle le composé de formule (I) avec de bons rendements à partir d'une matière première simple et peu onéreuse ;
- il permet d'éviter une réaction d'aromatisation puisque le noyau naphtalénique est présent dans le substrat de départ ;
- il permet d'obtenir l'agomélatine à partir du 7-méthoxy-naphtalén-2-ol avec un nombre réduit d'étapes.

Les composés de formule (V) obtenus selon le procédé de l'invention sont nouveaux et utiles en tant qu'intermédiaire de synthèse de l'agomélatine et du composé de formule (I).

Les composés de formule (V) préférés sont les suivants :
- 4-méthylbenzènesulfonate de 1-formyl-7-méthoxynaphtalén-2-yle ;
- méthanesulfonate de 1-formyl-7-méthoxynaphtalén-2-yle.

Le composé de formule (I) ainsi obtenu est, le cas échéant, soumis à une série de réactions chimiques classiques (par exemple : réduction de l'aldéhyde en alcool primaire, cyanation, réduction et acétylation de l'amine primaire obtenue) pour conduire à l'agomélatine de formule (II).

Les exemples ci-dessous illustrent l'invention, mais ne la limitent en aucune façon.
Afin de bien valider les chemins réactionnels, les intermédiaires de synthèse ont été systématiquement isolés et caractérisés. Toutefois, il est possible d'optimiser considérablement les procédés en limitant le nombre d'intermédiaires isolés.

Les structures des composés décrits ont été confirmées par les techniques spectroscopiques usuelles : RMN du proton (s = singulet ; d = doublet ; dd = doublet dédoublé) ; RMN du carbone (s = singulet ; d = doublet ; q = quadruplet).

### EXEMPLE 1 : 7-méthoxynaphtalène-1-carbaldéhyde

### Stade A : 2-hydroxy-7-méthoxynaphtalène-1-carbaldéhyde

Dans un ballon équipé d'un réfrigérant, sont introduits le 7-méthoxy-naphtalén-2-ol (3,5 g ; 20,11 mmol), l'orthoformiate d'éthyle (3,51 mL ; 21,12 mmol) et l'aniline (1,83 mL ; 20,11 mmol). Après agitation pendant 20 heures au reflux et refroidissement, le solide est broyé dans une solution éthanolique d'acide chlorhydrique 2 M (20 mL). Au bout de 30 minutes d'agitation à 60 °C et refroidissement, le solide est collecté par filtration pour être ensuite lavé à l'eau et séché par azéotropie avec de l'éthanol et utilisé directement sans autre purification (2,95 g ; 73 %).
*Analyse spectroscopique de RMN ¹H (CDCl₃, δ en ppm) : 13,17 (s, 1H) ; 10,74 (s*, *1H) ; 7,88 (d, J* = *9,1 Hz, 1H) ; 7,69 (d, J = 8,9 Hz, 1H) ; 7,65 (d*, *J* = *2,4 Hz, 1H) ; 7,07 (dd*, *J* = *8,9 et 2,4 Hz, 1H) ; 6*,*97 (d, J* = *9,1 Hz, 1H) ; 3,95 (s, 3H).*

### Stade B : 4-méthylbenzènesulfonate de 1-formyl-7-méthoxynaphtalén-2-yle

A une solution du produit du Stade A précédent (1 g ; 4,95 mmol) dans le dichlorométhane (20 mL), sont ajoutés la triéthylamine (826 µL ; 5,94 mmol) et le chlorure de tosyle (0,99 g ; 5,2 mmol). Au bout de 24 heures d'agitation, le solvant est évaporé puis le résidu est repris dans un mélange eau / acétate d'éthyle. La phase organique est lavée avec une solution diluée d'acide chlorhydrique, de l'eau et de la saumure, puis séchée sur sulfate de sodium et filtrée. L'évaporation des solvants fournit un brut qui est purifié par recristallisation dans l'acétate d'éthyle chaud pour conduire au produit du titre (1,132 g ; 65 %).
*Point de fusion : 147-148 °C.*
*Analyse spectroscopique de RMN ¹H (CDCl₃, δ en ppm) : 10,41 (s, 1H) ; 8,68 (d, J* = *2,6 Hz, 1H) ; 7,95 (d, J* = *8, 9 Hz, 1H) ; 7,74 (d, J* = *8,2 Hz, 2H) ; 7,72 (d, J* = *8,9 Hz, 1H) ; 7,33 (d, J* = *8,2 Hz, 2H) ; 7,19 (dd, J = 8, 9 et 2,6 Hz, 1H) ; 7,15 (d, J = 8, 9 Hz, 1H) ; 3,93 (s, 3H) ; 2,45 (s*, *3H).*
*Analyse spectroscopique de RMN ¹³C (CDCl₃, δ en ppm) : 190,3 (d) ; 161*,*5 (s) ; 154,3 (s) ; 146,4 (s) ; 136*,*4 (d) ; 132,8 (s) ; 131,5 (s) ; 130,3 (2 x d) ; 129,9 (d) ; 128,6 (2 x d) ; 127,8 (s) ; 121, 5 (s) ; 120,1 (d) ; 118,6 (d) ; 104,1 (d) ; 55, 6 (q) ; 21, 9 (q).*

### Stade C : 7-méthoxynaphtalène-1-carbaldéhyde

Dans un ballon mis à l'étuve et purgé à l'argon, sont introduits le produit du Stade B précédent (356 mg ; 1 mmol), l'acétate de palladium (4,5 mg ; 0,02 mmol), le 1,3-bis(diphénylphosphino)propane (8,2 mg ; 0,02 mmol), le diméthylformamide (2 mL), la triéthylamine (556 µL ; 4 mmol) et l'acide formique (150 µL ; 4 mmol). Le flacon est placé dans un bain chauffé à 90 °C pendant 1,5 heures. Après refroidissement, le mélange est dilué dans l'acétate d'éthyle et la phase organique est lavé avec une solution aqueuse d'acide chlorhydrique 1 M et avec de la saumure, séchée sur sulfate de sodium et filtrée. Après évaporation du solvant, le brut est purifié par filtration sur alumine neutre pour donner le produit du titre (139 mg ; 75 %).
*Point de fusion : 65-67 °C.*
*Analyse spectroscopique de RMN ¹H (CDCl₃, 300,13 MHz, δ en ppm) : 10, 29 (s, 1H) ; 8,75 (d, J = 2*,*6 Hz, 1H) ; 7*,*99 (d, J* = *8,1 Hz, 1H) ; 7, 9 (d, J* = *7,1 Hz, 1H) ; 7,77 (d, J* = *8,9 Hz, 1H) ; 7,45 (dd, J* = *8,1 et 7,1 Hz, 1H) ; 7, 23 (dd, J = 8, 9 et 2, 6 Hz, 1H) ; 3,98 (s, 3H).*
*Analyse spectroscopique de RMN ¹³C (CDCl₃, 75,5 Mhz, δ en ppm) : 194,1 (d) ; 160,7 (s) ; 138,3 (d) ; 135,1 (d) ; 132, 2 (s) ; 130, 2 (s) ; 129,9 (d) ; 129,3 (s) ; 122,5 (d) ; 119,8 (d) ; 103, 6 (d) ; 55, 6 (q).*

### EXEMPLE 2 : 7-méthoxynaphtalène-1-carbaldéhyde

### Stade A : méthanesulfonate de 1-formyl-7-méthoxynaphtalén-2-yle

A une solution du composé obtenu au Stade A de l'Exemple 1 (300 mg ; 1,485 mmol) dans le dichlorométhane (5 mL), sont ajoutés la triéthylamine (250 µL ; 1,782 mmol) et le chlorure de mésyle (120 µL). Après une heure d'agitation, le solvant est évaporé et le résidu est repris dans un mélange acétate d'éthyle / eau. La fraction organique est lavée deux fois à l'eau puis avec de la saumure, séchée sur sulfate de sodium et filtrée. L'évaporation du solvant donne le produit du titre propre (416 mg ; 95 %) sans besoin de purification.
*Analyse spectroscopique de RMN ¹H (CDCl₃, δ en ppm) : 10, 74 (s, 1H) ; 8, 72 (d, J = 2*,*4 Hz, 1H) ; 8,03 (d, J* = *8,9 Hz, 1H) ; 7,75 (d, J* = *8,9 Hz, 1H) ; 7, 3 6 (d, J* = *8,9 Hz, 1H) ; 7, 22 (dd, J* = *8,9 et 2,4 Hz, 1H) ; 3,97 (s*, *3H) ; 3,32 (s, 3H).*
*Analyse spectroscopique de RMN ¹³C (CDCl₃, δ en ppm) : 190,4 (d) ; 161,6 (s) ; 153,2 (s) ; 136,8 (d) ; 133,1 (s) ; 130,0 (d) ; 128,0 (s) ; 121, 6 (s) ; 120,3 (d) ; 118,2 (d) ; 104,0 (d) ; 55,7 (q) ; 38,5 (q).*

### Stade B : 7-méthoxynaphtalène-1-carbaldéhyde

Le produit du titre (84 %) est obtenu selon le procédé décrit dans le Stade C de l'Exemple 1 à partir du produit du Stade A précédent et un temps de réaction de 4 heures à 90 °C au lieu de 1,5 heures.
*Point de fusion : 65-67 °C.*
*Analyse spectroscopique de RMN ¹H (CDCl₃, 300,13 MHz, δ en ppm) : 10,29 (s, 1H) ; 8*,*75 (d, J* = *2,6 Hz, 1H) ; 7,99 (d, J = 8,1 Hz, 1H) ; 7,9 (d, J* = *7,1 Hz, 1H) ; 7,77 (d, J* = *8,9 Hz, 1H) ; 7,45 (dd, J* = *8,1 et 7,1 Hz, 1H) ; 7, 23 (dd, J* = *8,9 et 2,6 Hz, 1H) ; 3,98 (s, 3H).*
*Analyse spectroscopique de RMN ¹³C (CDCl₃, 75,5 MHz, δ en ppm) : 194,1 (d) ; 160,7 (s) ; 138,3 (d) ; 135,1 (d) ; 132,2 (s) ; 130,2 (s) ; 129,9 (d) ; 129,3 (s) ; 122,5 (d) ; 119,8 (d) ; 103,6 (d) ; 55,6 (q).*

### EXEMPLE 3 : 7-méthoxynaphtalène-1-carbaldéhyde

Dans un ballon purgé à l'argon, à une solution de 7-méthoxy-naphtalén-2-ol (70 mg ; 0,35 mmol) dans le diméthylformamide anhydre (1 mL), est ajouté en plusieurs portions l'hydrure de sodium (60 % ; 17 mg ; 0,415 mmol). Au bout de 30 minutes d'agitation à température ambiante, le chlorure de tosyle est alors ajouté en plusieurs portions (190,5 mg; 0,36 mmol). Après 4 heures d'agitation à température ambiante, le 1,3-bis(diphénylphosphino)propane (7,1 mg ; 0,017 mmol), l'acétate de palladium (3,9 mg ; 0,073 mmol), la triéthylamine (192 µL ; 1,38 mmol) et l'acide formique (150 µL ; 4 mmol) sont ajoutés et le mélange réactionnel est chauffé à 90 °C pendant 1,5 heures. Après refroidissement, le mélange est dilué dans l'acétate d'éthyle et la phase organique est lavée avec une solution aqueuse d'acide chlorhydrique 1 M puis avec de la saumure, séchée sur sulfate de sodium et filtrée. Après évaporation du solvant, le brut est filtré sur alumine neutre (éluant : acétate d'éthyle) pour donner le produit du titre (61,6 mg ; 95 %).
*Point de fusion : 65-67 °C.*

## Revendications

1. Procédé de synthèse industriel du composé de formule (I) : 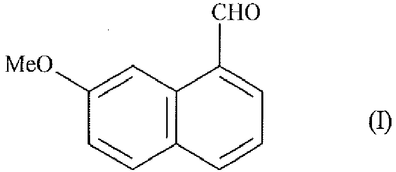 **caractérisé en ce que** l'on met en réaction le 7-méthoxy-naphtalén-2-ol de formule (III) : 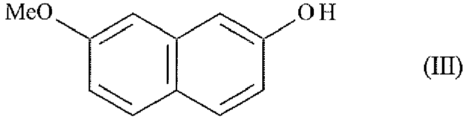 sur lequel on introduit sur la position 1 le groupement formyle pour conduire au composé de formule (IV) : 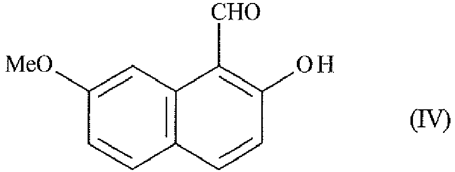 composé de formule (IV) qui est soumis à une réaction de sulfonylation pour conduire au composé de formule (V) : 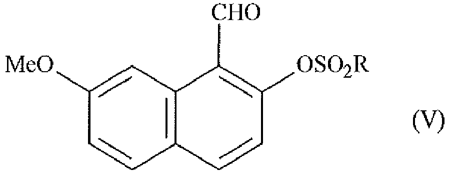 dans laquelle R représente un groupement -CH₃, -(CH₂)₂-CH₃, -CF₃ ou toluyle ; composé de formule (V) qui subit une réaction de désoxygénation en présence d'un métal de transition et d'un agent réducteur pour conduire au composé de formule (I) que l'on isole sous forme d'un solide.

2. Procédé de synthèse industriel du composé de formule (I) selon la revendication 1 **caractérisé en ce que** R représente un groupement -CH₃ ou toluyle.

3. Procédé de synthèse industriel du composé de formule (I) selon la revendication 1 **caractérisé en ce que** la transformation du composé de formule (IV) en composé de formule (V) est réalisée par action d'un chlorure de sulfonyle, d'un anhydride sulfonique ou d'un sulfonimide.

4. Procédé de synthèse industriel du composé de formule (I) selon la revendication 3 **caractérisé en ce que** la transformation du composé de formule (IV) en composé de formule (V) est réalisée par action d'un chlorure de sulfonyle.

5. Procédé de synthèse industriel du composé de formule (I) selon la revendication 1 **caractérisé en ce que**, lors de la transformation du composé de formule (V) en composé de formule (I), le métal de transition est le nickel, le palladium ou le platine.

6. Procédé de synthèse industriel du composé de formule (I) selon la revendication 1 **caractérisé en ce que**, lors de la transformation du composé de formule (V) en composé de formule (I), le métal de transition est un sel de palladium.

7. Procédé de synthèse industriel du composé de formule (I) selon la revendication 1 **caractérisé en ce que** la transformation du composé de formule (V) en composé de formule (I) est réalisée dans le diméthylformamide, le dioxane, le tétrahydrofurane ou le toluène.

8. Procédé de synthèse industriel du composé de formule (I) selon la revendication 7 **caractérisé en ce que** la transformation du composé de formule (V) en composé de formule (I) est réalisée dans le diméthylformamide.

9. Procédé de synthèse industriel du composé de formule (I) selon la revendication 1 **caractérisé en ce que** la transformation du composé de formule (V) en composé de formule (I) est réalisée entre 25 °C et 110 °C.

10. Procédé de synthèse industriel du composé de formule (I) selon la revendication 9 **caractérisé en ce que** la transformation du composé de formule (V) en composé de formule (I) est réalisée entre 40 °C et 95 °C.

11. Procédé de synthèse industriel du composé de formule (I) selon la revendication 1 **caractérisé en ce que**, lors de la transformation du composé de formule (V) en composé de formule (I), l'agent réducteur est le dihydrogène.

12. Procédé de synthèse industriel du composé de formule (I) selon la revendication 11 **caractérisé en ce que** le dihydrogène est obtenu par décomposition d'un formiate d'ammonium.

13. Procédé de synthèse industriel du composé de formule (I) selon la revendication 1 **caractérisé en ce que** la transformation du composé de formule (V) en composé de formule (I) est réalisée en présence de palladium et de dihydrogène.

14. Procédé de synthèse industriel du composé de formule (I) selon la revendication 1 **caractérisé en ce que** la transformation du composé de formule (V) en composé de formule (I) est réalisée en présence de (9,9-diméthyl-9*H*-xanthène-4,5-diyl)bis(diphénylphosphane) ou de 1,3-bis(diphénylphosphino)propane.

15. Composé de formule (V) selon la revendication 1 utile en tant qu'intermédiaire de synthèse du composé de formule (I).

16. Composé de formule (V) selon la revendication 15 utile en tant qu'intermédiaire de synthèse de l'agomélatine de formule (II).

17. Composé de formule (V) selon les revendications 15 et 16 choisi parmi les composés suivants :
- 4-méthylbenzènesulfonate de 1-formyl-7-méthoxynaphtalén-2-yle ;
- méthanesulfonate de 1-formyl-7-méthoxynaphtalén-2-yle.

18. Utilisation du composé de formule (V) selon les revendications 15 à 17 dans la synthèse du composé de formule (I).

19. Utilisation du composé de formule (V) selon la revendication 18 dans la synthèse de l'agomélatine de formule (II).

20. Utilisation du composé de formule (III) selon la revendication 1 dans la synthèse du composé de formule (I).

21. Utilisation du composé de formule (III) selon la revendication 20 dans la synthèse de l'agomélatine de formule (II).

22. Procédé de synthèse de l'agomélatine à partir du composé de formule (V) **caractérisé en ce que** le composé de formule (V) est obtenu par le procédé de synthèse selon l'une des revendications 1 à 4.

## Patentansprüche

1. Verfahren zur industriellen Synthese der Verbindung der Formel (I): 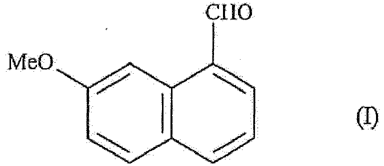 **dadurch gekennzeichnet, dass** man 7-Methoxy-naphthalin-2-ol der Formel (III): 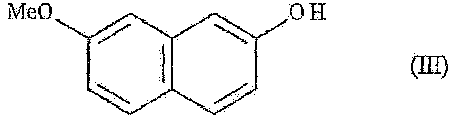 zur Einführung der Formylgruppe in ihre Stellung 1 umsetzt zur Bildung der Verbindung der Formel (IV): 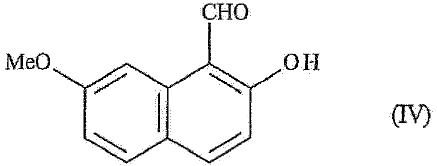 welche Verbindung der Formel (IV) einer Sulfonylierungsreaktion unterworfen wird zur Bildung der Verbindung der Formel (V): 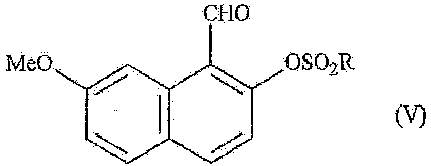 in der R eine Gruppe -CH₃, -(CH₂)₂-CH₃, -CF₃ oder eine Toluylgruppe bedeutet, welche Verbindung der Formel (V) einer Desoxygenierungsreaktion in Gegenwart eines Übergangsmetalls und eines Reduktionsmittels unterworfen wird zur Bildung der Verbindung der Formel (I), welche man in Form eines Feststoffs isoliert.

2. Verfahren zur industriellen Synthese der Verbindung der Formel (I) nach Anspruch 1, **dadurch gekennzeichnet, dass** R eine -CH₃- oder eine Toluylgruppe bedeutet.

3. Verfahren zur industriellen Synthese der Verbindung der Formel (I) nach Anspruch 1, **dadurch gekennzeichnet, dass** die Umwandlung der Verbindung der Formel (IV) in die Verbindung der Formel (V) durch Einwirkung von Sulfonylchlorid, eines Sulfonsäureanhydrids oder eines Sulfonimids durchgeführt wird.

4. Verfahren zur industriellen Synthese der Verbindung der Formel (I) nach Anspruch 3, **dadurch gekennzeichnet, dass** die Umwandlung der Verbindung der Formel (IV) in die Verbindung der Formel (V) durch Einwirkung eines Sulfonylchlorids durchgeführt wird.

5. Verfahren zur industriellen Synthese der Verbindung der Formel (I) nach Anspruch 1, **dadurch gekennzeichnet, dass** das bei der Umwandlung der Verbindung der Formel (V) in die Verbindung der Formel (I) eingesetzte Übergangsmetall Nickel, Palladium oder Platin ist.

6. Verfahren zur industriellen Synthese der Verbindung der Formel (I) nach Anspruch 1, **dadurch gekennzeichnet, dass** das bei der Umwandlung der Verbindung der Formel (V) in die Verbindung der Formel (I) eingesetzte Übergangsmetall ein Palladiumsalz ist.

7. Verfahren zur industriellen Synthese der Verbindung der Formel (I) nach Anspruch 1, **dadurch gekennzeichnet, dass** die Umwandlung der Verbindung der Formel (V) in die Verbindung der Formel (I) in Dimethylformamid, Dioxan, Tetrahydrofuran oder Toluol durchgeführt wird.

8. Verfahren zur industriellen Synthese der Verbindung der Formel (I) nach Anspruch 7, **dadurch gekennzeichnet, dass** die Umwandlung der Verbindung der Formel (V) in die Verbindung der Formel (I) in Dimethylformamid durchgeführt wird.

9. Verfahren zur industriellen Synthese der Verbindung der Formel (I) nach Anspruch 1, **dadurch gekennzeichnet, dass** die Umwandlung der Verbindung der Formel (V) in die Verbindung der Formel (I) zwischen 25 °C und 110 °C durchgeführt wird.

10. Verfahren zur industriellen Synthese der Verbindung der Formel (I) nach Anspruch 9, **dadurch gekennzeichnet, dass** die Umwandlung der Verbindung der Formel (V) in die Verbindung der Formel (I) zwischen 40 °C und 95 °C durchgeführt wird.

11. Verfahren zur industriellen Synthese der Verbindung der Formel (I) nach Anspruch 1, **dadurch gekennzeichnet, dass** das bei der Umwandlung der Verbindung der Formel (V) in die Verbindung der Formel (I) eingesetzte Reduktionsmittel Wasserstoff ist.

12. Verfahren zur industriellen Synthese der Verbindung der Formel (I) nach Anspruch 11, **dadurch gekennzeichnet, dass** der Wasserstoff durch Zersetzung eines Ammoniumformiats erhalten wird.

13. Verfahren zur industriellen Synthese der Verbindung der Formel (I) nach Anspruch 1, **dadurch gekennzeichnet, dass** die Umwandlung der Verbindung der Formel (V) in die Verbindung der Formel (I) in Gegenwart von Palladium und Wasserstoff durchgeführt wird.

14. Verfahren zur industriellen Synthese der Verbindung der Formel (I) nach Anspruch 1, **dadurch gekennzeichnet, dass** die Umwandlung der Verbindung der Formel (V) in die Verbindung der Formel (I) in Gegenwart von (9,9-Dimethyl-9H-xanthen-4,5-diyl)-bis(diphenylphosphan) oder 1,3-Bis(diphenylphosphino)-propan durchgeführt wird.

15. Verbindung der Formel (V) nach Anspruch 1, nützlich als Zwischenprodukt bei der Synthese der Verbindung der Formel (I).

16. Verbindung der Formel (V) nach Anspruch 15, nützlich als Zwischenprodukt bei der Synthese von Agomelatin der Formel (II).

17. Verbindung der Formel (V) nach den Ansprüchen 15 und 16, ausgewählt aus den folgenden Verbindungen:
- 1-Formyl-7-methoxynaphthalin-2-yl-4-methylbenzolsulfonat;
- 1-Formyl-7-methoxynaphthalin-2-yl-methansulfonat.

18. Verwendung der Verbindung der Formel (V) nach den Ansprüchen 15 bis 17 bei der Synthese der Verbindung der Formel (I).

19. Verwendung der Verbindung der Formel (V) nach Anspruch 18 bei der Synthese von Agomelatin der Formel (II).

20. Verwendung der Verbindung der Formel (III) nach Anspruch 1 bei der Synthese der Verbindung der Formel (I).

21. Verwendung der Verbindung der Formel (III) nach Anspruch 20 bei der Synthese von Agomelatin der Formel (II).

22. Verfahren zur Synthese von Agomelatin ausgehend von der Verbindung der Formel (V), **dadurch gekennzeichnet, dass** die Verbindung der Formel (V) erhalten wird nach dem Syntheseverfahren gemäß einem der Ansprüche 1 bis 4.

## Claims

1. Process for the industrial synthesis of the compound of formula (I): 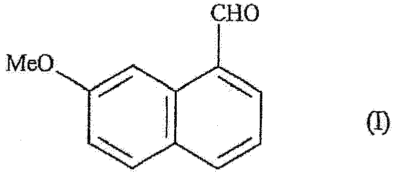 **characterised in that** 7-methoxy-naphthalen-2-ol of formula (III): 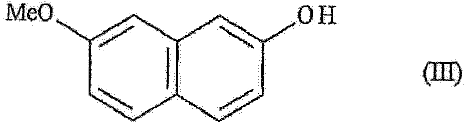 is reacted, wherein a formyl group is introduced at position 1 to yield the compound of formula (IV): 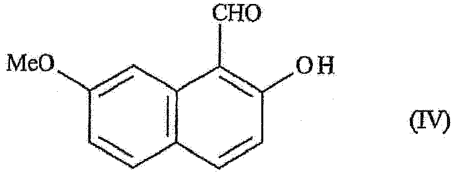 which compound of formula (IV) is subjected to a sulfonylation reaction to yield the compound of formula (V): 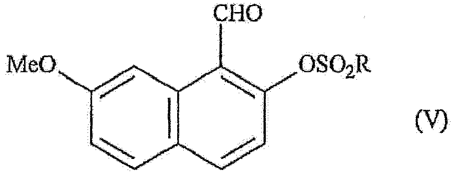 wherein R represents a group -CH₃, -(CH₂)₂-CH₃, -CF₃ or toluyl; which compound of formula (V) is subjected to a deoxygenation reaction in the presence of a transition metal and a reducing agent to yield the compound of formula (I), which is isolated in the form of a solid.

2. Process for the industrial synthesis of the compound of formula (I) according to claim 1, **characterised in that** R represents a group -CH₃ or toluyl.

3. Process for the industrial synthesis of the compound of formula (I) according to claim 1, **characterised in that** the conversion of the compound of formula (IV) into the compound of formula (V) is carried out by the action of a sulfonyl chloride, a sulfonic anhydride or a sulfonamide.

4. Process for the industrial synthesis of the compound of formula (I) according to claim 3, **characterised in that** the conversion of the compound of formula (IV) into the compound of formula (V) is carried out by the action of a sulfonyl chloride.

5. Process for the industrial synthesis of the compound of formula (I) according to claim 1, **characterised in that**, in the conversion of the compound of formula (V) into the compound of formula (I), the transition metal is nickel, palladium or platinum.

6. Process for the industrial synthesis of the compound of formula (I) according to claim 1, **characterised in that**, in the conversion of the compound of formula (V) into the compound of formula (I), the transition metal is a palladium salt.

7. Process for the industrial synthesis of the compound of formula (I) according to claim 1, **characterised in that** the conversion of the compound of formula (V) into the compound of formula (I) is carried out in dimethylformamide, dioxane, tetrahydrofuran or toluene.

8. Process for the industrial synthesis of the compound of formula (I) according to claim 7, **characterised in that** the conversion of the compound of formula (V) into the compound of formula (I) is carried out in dimethylformamide.

9. Process for the industrial synthesis of the compound of formula (I) according to claim 1, **characterised in that** the conversion of the compound of formula (V) into the compound of formula (I) is carried out between 25°C and 110°C.

10. Process for the industrial synthesis of the compound of formula (I) according to claim 9, **characterised in that** the conversion of the compound of formula (V) into the compound of formula (I) is carried out between 40°C and 95°C.

11. Process for the industrial synthesis of the compound of formula (I) according to claim 1, **characterised in that**, in the conversion of the compound of formula (V) into the compound of formula (I), the reducing agent is dihydrogen.

12. Process for the industrial synthesis of the compound of formula (I) according to claim 11, **characterised in that** the dihydrogen is obtained by decomposition of an ammonium formate.

13. Process for the industrial synthesis of the compound of formula (I) according to claim 1, **characterised in that** the conversion of the compound of formula (V) into the compound of formula (I) is carried out in the presence of palladium and dihydrogen.

14. Process for the industrial synthesis of the compound of formula (I) according to claim 1, **characterised in that** the conversion of the compound of formula (V) into the compound of formula (I) is carried out in the presence of (9,9-dimethyl-9*H*-xanthene-4,5-diyl)bis(diphenylphosphane) or 1,3-bis(di-phenylphosphino)propane.

15. Compound of formula (V) according to claim 1 for use as an intermediate for the synthesis of the compound of formula (I).

16. Compound of formula (V) according to claim 15 for use as an intermediate for the synthesis of agomelatine of formula (II).

17. Compound of formula (V) according to claims 15 and 16 chosen from the following compounds:
- 1-formyl-7-methoxynaphthalen-2-yl 4-methylbenzenesulfonate;
- 1-formyl-7-methoxynaphthalen-2-yl methanesulfonate.

18. Use of the compound of formula (V) according to claims 15 to 17 in the synthesis of the compound of formula (I).

19. Use of the compound of formula (V) according to claim 18 in the synthesis of agomelatine of formula (II).

20. Use of the compound of formula (III) according to claim 1 in the synthesis of the compound of formula (I).

21. Use of the compound of formula (III) according to claim 20 in the synthesis of agomelatine of formula (II).

22. Process for the synthesis of agomelatine from the compound of formula (V), **characterised in that** the compound of formula (V) is obtained by the synthesis process according to any one of claims 1 to 4.
